# EUROPÄISCHE PATENTSCHRIFT

(11) **EP 0 609 752 B1**
(45) Veröffentlichungstag und Bekanntmachung des Hinweises auf die Patenterteilung: **02.04.1997**
(21) Anmeldenummer: 94101031.6
(22) Anmeldetag: 25.01.1994
(51) Int. Cl.: C07D 207/267, C07D 201/08

(54) **Verfahren zur Herstellung von N-substituierten Pyrrolid-2-onen**
Process for the preparation of N-substituted 2-pyrrolidones
Procédé pour la préparation de 2-pyrrolidones N-substituées

(30) Priorität: 05.02.1993 DE 4303334
(43) Veröffentlichungstag der Anmeldung: 10.08.1994
(73) Patentinhaber: BASF Aktiengesellschaft, 67063 Ludwigshafen (DE)
(72) Erfinder: Weyer, Hans-Juergen, Dr., D-68167 Mannheim (DE); Fischer, Rolf, Dr., D-69121 Heidelberg (DE)

(56) Entgegenhaltungen:
- EP-A- 0 460 474
- US-A- 3 448 118

## Beschreibung

Die vorliegende Erfindung betrifft ein verbessertes Verfahren zur Herstellung von N-substituierten Pyrrolid-2-onen (I) durch Umsetzung von Maleinsäure, Fumarsäure oder Bernsteinsäure oder funktionellen Derivaten dieser Säuren (Verbindungen II) mit einem primären Amin (III) oder von Amiden oder Imiden aus II und III unter hydrierenden Bedingungen in einer Synthesestufe und destillative Gewinnung von I und sonstigen flüchtigen Bestandteilen aus dem so erhaltenen Reaktionsgemisch.

Es ist bekannt, N-substituierte Pyrrolid-2-one aus einer Vielzahl von Verbindungen durch katalytische Hydrierung herzustellen. Bei den Ausgangsverbindungen handelt es sich um Gemische aus einem primären Amin (II) und Maleinsäureanhydrid (DE-A 40 18 243, US-A 3 109 005) oder Fumarsäure, Maleinsäure, Bernsteinsäure, funktionelle Derivate dieser Säuren wie Bernsteinsäureanhydrid oder Amide und Imide aus II und den genannten Säuren, die alle zu N-substituierten Pyrrolid-2-onen hydriert werden können. Unabhängig von der Art dieser Ausgangsstoffe werden die nach der Hydrierung erhaltenen Reaktionsgemische destillativ aufgearbeitet. Aufgrund ihrer Flüchtigkeit können die gewünschten Pyrrolidone, die leichter flüchtigen Pyrrolidine und die weniger flüchtigen Bernsteinsäureimide destillativ getrennt werden. Unter den Destillationsbedingungen cyclisieren offenkettige Bernsteinsäureamide zu cyclischen Imiden und können somit ebenfalls abgetrennt werden. Nach der Destillation verbleibt ein hochsiedender Rückstand. Dieser Rückstand, der bisher verbrannt wurde, entspricht in der Regel einigen Mol-% der Ausgangsverbindungen; unter Hydrierbedingungen, die besonders selektiv zu den entsprechenden Pyrrolidonen führen, kann diese Rückstandsmenge aber beträchtlich größer sein.

Es bestand die Aufgabe, ein Verfahren bereitzustellen, das es erlaubt, aus diesen hochsiedenden Destillationsrückständen Wertprodukte zu gewinnen.

Demgemäß wurde das oben definierte Verfahren gefunden, das dadurch gekennzeichnet ist, daß man den nach der Destillation anfallenden Rückstand einer nochmaligen hydrierenden Behandlung unterwirft und das hierbei entstehende (I) destillativ gewinnt.

Die erfindungsgemäß zu verwendenden hochsiedenden Destillationsrückstände sind üblicherweise solche Produkte, die bei einem Druck von ca. 10 mbar bis zu Temperaturen von 200°C nicht flüchtig sind. Vermutlich handelt es sich bei diesen Produkten um oligomere, gegebenenfalls substituierte Bernsteinsäurediamide.

Diese Destillationsrückstände werden einer erneuten Hydrierung unterworfen. Die Hydrierung erfolgt an üblichen Hydrierkatalysatoren, wobei die katalytisch aktive Komponente ein Metall der Gruppe 8 bis 10 des Periodensystems, Chrom, Mangan, Rhenium oder Kupfer enthält.

Die verwendeten Katalysatoren können im erfindungsgemäßen Verfahren sowohl als Trägerkatalysatoren oder in kompakter Form, d.h. ohne Träger eingesetzt werden. Es können übliche Trägermaterialien wie Aktivkohle, Siliciumdioxid, Aluminiumoxide, Titandioxide, Zirkonoxid, Silikate oder Zeolithe verwendet werden.

Die Katalysatoren werden vorzugsweise vor ihrem Einsatz im erfindungsgemäßen Verfahren mit Wasserstoff aktiviert. Dabei werden die nach der Calcinierung im allgemeinen in Form ihrer Oxide vorliegenden aktiven Katalysatorbestandteile größtenteils reduziert, und zwar in der Regel zu den entsprechenden Metallen.

Die Katalysatoren können als Suspensions- oder Festbettkatalysatoren eingesetzt werden. Die Reaktion kann in Rührkesseln oder Rohrreaktoren durchgeführt werden. Werden Rohrreaktoren verwendet, können die Einsatzstoffe in der Sumpf- oder der Rieselfahrweise über den Katalysator geleitet werden. Die Reaktion kann sowohl kontinuierlich wie auch diskontinuierlich vorgenommen werden.

Der Wasserstoff kann in stöchiometrischen Mengen, vorzugsweise aber in überstöchiometrischen Mengen in die Reaktion geführt werden. Die Menge des zugeführten überschüssigen Wasserstoffs ist nicht kritisch, da dieser in die Umsetzung zurückgeführt werden kann.

Die hydrierende Umsetzung wird im allgemeinen bei Temperaturen von 100 bis 350°C, vorzugsweise 150 bis 300°C und insbesondere bei 160 bis 280°C vorgenommen. Dabei wird im allgemeinen bei Drucken von 50 bis 350 bar und bevorzugt bei 100 bis 300 bar gearbeitet.

Die erfindungsgemäße hydrierende Umsetzung der Destillationsrückstände wird im allgemeinen in Gegenwart eines Lösungsmittels ausgeführt. Als Lösungsmittel können praktisch alle Lösungsmittel verwendet werden, welche sich unter den Bedingungen der Hydrierung inert verhalten, beispielsweise Wasser, aliphatische und aromatische Kohlenwasserstoffe sowie Ether wie Diethylether, Diisopropylether, Methyl-tert.-Butylether, Dioxan und Tetrahydrofuran oder Gemische dieser Lösungsmittel. Vorteilhaft ist der Einsatz von N-substituierten Pyrrolid-2-onen als Lösungsmittel, wie sie bei der erfindungsgemäßen Umsetzung entstehen. Besonders bevorzugt ist Wasser, insbesondere für die Herstellung von N-Methylpyrrolidon. Es ist aber auch möglich, die Destillationsrückstände als Schmelze in die Hydrierung zu dosieren.

In bezug auf die Substituenten am Stickstoffatom des N-substituierten Pyrrolid-2-ons unterliegt das Verfahren keinen erkennbaren Einschränkungen. Die Ausgangsgemische können als primäre Amine III beispielsweise aliphatische Amine mit 1 bis 10, vorzugsweise 1 bis 4 Kohlenstoffatomen, primäre cycloaliphatische Amine mit 5 bis 8 Kohlenstoffatomen, aber auch primäre aromatische und araliphatische Amine wie Anilin oder Benzylamin enthalten. Im Hinblick auf die Bedeutung der Verfahrensprodukte sind hier in erster Linie Propylamin, Butylamin, Hexylamin, Decylamin, Cyclopentylamin und Cyclohexylamin zu nennen, sowie vor allem Methylamin.

Die Hydrierung kann in Abwesenheit, bevorzugt aber in Anwesenheit des bei der Herstellung der Destillationsrückstände verwendeten Amins stattfinden. In einer bevorzugten Variante des erfindungsgemäßen Verfahrens zur Hydrierung der Destillationsrückstände werden diese in die Synthesestufe zurückgeführt.

Eine weitere Ausführungsform der vorliegenden Erfindung besteht darin, die hochsiedenden Destillationsrückstände einer Thermolyse zu unterwerfen, indem man sie zunächst einer höheren Temperatur als der Destillationstemperatur aussetzt und die hierbei erhaltenen flüchtigen Produkte anschließend wie oben beschrieben hydriert. Weiterhin bietet die Thermolyse die Möglichkeit, die als Hauptprodukte gebildeten N-substituierten Bernsteinsäureimide zu isolieren und in die Synthesestufe zurückzuführen. Die Thermolyse der Destillationsrückstände ist besonders dann zu empfehlen, wenn es bei der Rückführung des Destillationsrückstände in die Hydrierstufe zur Anreicherung von unerwünschten Nebenprodukten kommt.

Die Thermolyse wird im allgemeinen bei Temperaturen von 200 bis 500°C, vorzugsweise bei 250 bis 400°C und besonders bevorzugt bei 280 bis 350°C vorgenommen. Dabei wird im allgemeinen bei Drucken unter 1 bar, bevorzugt bei 0,01 bis 100 mbar und insbesondere bei 1 bis 50 mbar gearbeitet.

Die Austräge, die durch die erfindungsgemäße Hydrierung von Destillationsrückständen erhalten werden, zeigen eine vergleichbare Produktzusammensetzung, wie man sie bei der Hydrierung der Ausgangsverbindungen findet.

Die vorliegende Erfindung erlaubt die Überführung von hochsiedenden Destillationsrückständen in N-substituierte Pyrrolidone in guter Ausbeute. Die N-substituierten Pyrrolidone, insbesondere N-Methylpyrrolidon, werden hauptsächlich als Lösungs- und Extraktionsmittel verwendet.

### Beispiele

### Destillationsrückstand

Der hochsiedende Destillationsrückstand, der in den folgenden Beispielen eingesetzt wurde, wurde folgendermaßen erhalten:

Eine wäßrige Lösung aus Maleinsäureanhydrid und Methylamin (20 Gew.-% Maleinsäureanhydrid-Methylamin, Molverhältnis 1 : 1,5) wurde an einem Kobaltkatalysator bei 250°C und 250 bar hydriert. Durch anschließende destillative Aufarbeitung des Hydrieraustrags wurden 71 % N-Methylpyrrolidon, 5 % N-Methylpyrrolidin und 6 % Bernsteinsäure-N-methylimid gewonnen. Es verblieben 17 % Rückstand (berechnet als Bernsteinsäure-N-methylamid).

Der eingesetzte Kobaltkatalysator (hergestellt nach dem Verfahren der DE-A 23 21 101 und DE-A 39 04 083) hatte folgende Zusammensetzung:
63,4 Gew.-% Kobalt, ber. als CoO
18,1 Gew.-% Kupfer, ber. als CuO
6,8 Gew.-% Mangan, ber. als Mn₃O₄
3,1 Gew.-% Molybdän, ber. als MoO₃
0,15 Gew.-% Natrium, ber. als Na₂O
3,3 Gew.-% Phosphorsäure

Die in den Beispielen verwendeten Hydrierkatalysatoren wurden vor ihrem Einsatz wie üblich mit Wasserstoff aktiviert.

### Beispiel 1

Die hydrierende Umsetzung wurde in einem Rohrreaktor, in dem sich 38 g des Kobaltkatalysators in einem Festbett befanden, durchgeführt. Der Katalysator wurde in Form von 2,5 bis 4 mm Splitt eingesetzt.

In Rieselfahrweise wurden dem Reaktor bei einem Gesamtdruck von 200 bar und einer Temperatur von 250°C 0,15 kg Destillationsrückstand/kg Katalysator und Stunde und 0,55 kg Wasser/kg Katalysator und Stunde, sowie 2500 1 (bei Normaldruck) Wasserstoff/kg Katalysator und Stunde zugeführt.

Die Ausbeute an N-Methylpyrrolidon betrug 81 %, daneben wurden 6 % N-Methylpyrrolidin und 7 % Bernsteinsäure-N-methylimid gefunden. Die Ausbeuten beziehen sich auf den als Bernsteinsäuremono-N-methylamid berechneten Destillationsrückstand.

### Beispiel 2

Die Hydrierung wurde in Analogie zu Beispiel 1 durchgeführt. Als Katalysator wurden 36 g eines Nickelkatalysators der Zusammensetzung 50 Gew.-% NiO, 17 Gew.-% CuO, 31 Gew.-% ZrO₂ und 2 Gew.-% MoO₃ (hergestellt nach der US-A 5 037 793) in Form von 4 mm-Strängen eingesetzt. Bei einem Gesamtdruck von 200 bar und einer Temperatur von 250°C wurden dem Reaktor 0,2 kg Destillationsrückstand/kg Katalysator und Stunde, 0,1 kg Methylamin/kg Katalysator und Stunde und 0,7 kg Wasser/kg Katalysator und Stunde sowie 2500 1 (bei Normaldruck) Wasserstoff/kg Katalysator und Stunde zugeführt.

Die Ausbeute an N-Methylpyrrolidon betrug 67 % (bezogen auf den Destillationsrückstand, berechnet als Bernsteinsäuremono-N-methylamid)

### Beispiel 3, Thermolyse

50 g Destillationsrückstand wurden bei 1 mbar Druck und 280°C eine Stunde lang thermolysiert. Die entstandenen flüchtigen Produkte wurden abdestilliert und kondensiert. Es verblieben im Sumpf 9,5 g Rückstand. Es wurden 24,5 g Bernsteinsäure-N-methylimid isoliert (Ausbeute 57 % bezogen auf den Destillationsrückstand, berechnet als Bernsteinsäuremono-N-methylamid), die der Synthesestufe zugeführt wurden.

## Patentansprüche

1. Verfahren zur Herstellung von N-substituierten Pyrrolid-2-onen (I) durch Umsetzung von Maleinsäure, Fumarsäure oder Bernsteinsäure oder funktionellen Derivaten dieser Säuren (Verbindungen II) mit einem primären Amin (III) oder von Amiden oder Imiden aus II und III unter hydrierenden Bedingungen in einer Synthesestufe und destillative Gewinnung von I und sonstigen flüchtigen Bestandteilen aus dem so erhaltenen Reaktionsgemisch, dadurch gekennzeichnet, daß man den nach der Destillation anfallenden Rückstand einer nochmaligen hydrierenden Behandlung unterwirft und das hierbei entstehende (I) destillativ gewinnt.

2. Verfahren nach Anspruch 1, dadurch gekennzeichnet, daß man den nach der Destillation anfallenden Rückstand in die Synthesestufe zurückführt.

3. Verfahren nach Anspruch 1, dadurch gekennzeichnet, daß man den nach der Destillation anfallenden Rückstand zunächst einer höheren Temperatur als bei der Destillation und anschließend einer nochmaligen hydrierenden Behandlung unterwirft.

4. Verfahren nach Anspruch 3, dadurch gekennzeichnet, daß man die bei der thermischen Behandlung gebildeten flüchtigen Stoffe abdestilliert und in die Synthesestufe zurückführt.

## Claims

1. A process for preparing N-substituted 2-pyrrolidones (I) by reaction of maleic acid, fumaric acid or succinic acid or functional derivatives of these acids (compounds II) with a primary amine (III) or of amides or imides of II and III under hydrogenating conditions in one synthesis stage and isolation of I and other volatile constituents by distillation from the resulting reaction mixture, wherein the residue left after distillation is subjected to another hydrogenating treatment, and the (I) produced thereby is isolated by distillation.

2. A process as claimed in claim 1, wherein the residue left after the distillation is returned to the synthesis stage.

3. A process as claimed in claim 1, wherein the residue left after the distillation is subjected first to a temperature above that in the distillation and subsequently to another hydrogenating treatment.

4. A process as claimed in claim 3, wherein the volatile substances formed in the thermal treatment are distilled out and returned to the synthesis stage.

## Revendications

1. Procédé de préparation de pyrrolid-2-ones N-substituées (I) par réaction d'acide maléique, d'acide fumarique, d'acide succinique ou de dérivés fonctionnels de ces acides (composés II) avec une amine primaire (III), ou d'amides ou imides de II et de III, dans des conditions d'hydrogénation dans une étape de synthèse, et par récupération par distillation de I et d'autres constituants volatils à partir du mélange réactionnel ainsi obtenu, caractérisé en ce que l'on soumet à un nouveau traitement d'hydrogénation le résidu obtenu à la suite de la distillation et on récupère par distillation le composé (I) ainsi formé.

2. Procédé selon la revendication 1, caractérisé en ce que l'on renvoie dans l'étape de synthèse le résidu obtenu à la suite de la distillation.

3. Procédé selon la revendication 1, caractérisé en ce que l'on soumet tout d'abord à une température plus élevée que lors de la distillation le résidu obtenu à la suite de la distillation, puis on le soumet à un nouveau traitement d'hydrogénation.

4. Procédé selon la revendication 3, caractérisé en ce que l'on chasse par distillation les substances volatiles formées lors du traitement thermique et on les renvoie dans l'étape de synthèse.
